Europäisches Patentamt

European Patent Office

Office européen des brevets

(11) Publication number: **0 111 060**
**B1**

# EUROPEAN PATENT SPECIFICATION

(45) Date of publication of patent specification: **19.08.87**

(51) Int. Cl.⁴: **A 61 B 17/36, A 61 N 5/06**

(21) Application number: **83108760.6**

(22) Date of filing: **06.09.83**

(54) **Ablative photodecomposition of organic biological material.**

(30) Priority: **09.12.82 US 448123**

(43) Date of publication of application:
**20.06.84 Bulletin 84/25**

(45) Publication of the grant of the patent:
**19.08.87 Bulletin 87/34**

(84) Designated Contracting States:
**AT BE CH DE FR GB IT LI NL SE**

(56) References cited:
**US-A-4 144 888**

**LASER FOCUS, vol. 18, no. 1, January 1982, pages 57-58, H.T.POWELL: "Excimers"**

**REV. DE PHYSIQUE APPLIQUEE, vol. 15, no. 9, September 1980, pages 1417-1426, Paris, FR; J.M. BRUNETAUD et al.: "Les applications therapeutiques des lasers"**

**Applied Physics Letters 41(6), 15 September 1982, pp.576 to 578**

(73) Proprietor: **International Business Machines Corporation**
**Old Orchard Road**
**Armonk, N.Y. 10504 (US)**

(72) Inventor: **Blum, Samuel Emil**
**18-1 Grenada Crescent**
**White Plains New York 10603 (US)**
Inventor: **Srinivasan, Rangaswamy**
**98 Cedar Lane**
**Ossining New York 10562 (US)**
Inventor: **Wynne, James Jeffrey**
**RD3, Crow Hill Road**
**Mt Kisco New York 10549 (US)**

(74) Representative: **Appleton, John Edward et al**
**IBM United Kingdom Limited Intellectual Property Department Hursley Park**
**Winchester Hampshire SO21 2JN (GB)**

Courier Press, Leamington Spa, England.

**Description**

Technical field

This invention relates to ablative photodecomposition of organic biological material using far ultraviolet radiation of wavelength less then 200 nm. Ablative photodecomposition is more fully discussed hereinafter. Briefly organic biological material is exposed to UV radiation of sufficient energy to break the constituent bonds of the material, resulting in volatile or gaseous products, typically of low molecular weight, which "explode" away from the material, so that it is photo-etched.

Background art

The use of radiation from lasers in medical and dental procedures has been known for some time, having been applied shortly after the invention of the laser in 1960. In 1961, medical researchers treated animal and human retinas and showed that a laser beam could induce a lesion on the retina for therapeutic purposes. Such laser eye surgery for detached retinas and other disorders is now routine in eye clinics throughout the world. In this application, and in others using laser beams, the laser beam is absorbed by the irradiated tissue causing heating, denaturing of protein, and tissue death. The results are therapeutic because of the formation of scar tissue, cauterization of the bleeding blood vessels, or the cutting away of diseased or damaged tissue. Thus, in prior art applications of intense laser radiation, the laser was used to provide a directed source of the radiation whose thermal energy led to the pyrolysis of the organic matter. However, there are many situations where heating is not desired and is in fact harmful, and in those situations such lasers may not be used. As will be more apparent from the following, the present invention is primarily applicable to a technique for using radiation in a manner in which unnecessary heating and damage to surrounding organic tissue is avoided.

- In the prior art, the intense laser radiation was generally in visible or infrared regions of the spectrum. For example, U.S. Patent 3,769,963 describes an instrument for performing laser micro-surgery and describes the use of lasers for ophthalmology, dermatology, and experimental surgery. In this patent, a great deal of background information about laser treatments is provided, and the preferred wavelengths of light are stated to be 300—1000 nm although selective absorption of energy is noted in the range 200—3000 nm. In particular, selective absorption is noted in the visible range of 400—700 nm and in the infrared range at 1000 nm and 2000 nm.

Laser treatment of skin defects and lesions is described in U.S. Patent 4,316,467 where a system is described for regulating the laser energy output in accordance with the absorption of the tissue being irradiated. Another reference describing lasers for medical and dental applications is U.S. Patent 3,821,510. This patent describes a flexible laser beam transmitting device which can be held by hand and has certain adjustment features.

U.S. Patent 4,273,535 describes a method for preventing tooth decay using gaint pulses produced from a laser having an output wavelength of 1.06 micrometers (1060 nm). In particular, a flexible glass fibre is used as a laser beam guide for directing the laser energy from the laser source to the area to be irradiated.

In the prior art, the selectivity in absorption of different types of tissues has been noted, but the wavelengths used have been 200 nm and longer. To prevent irradiation (and its consequent damage) to non-selected areas surrounding the area selected for irradiation, it might be thought to use a mask. Even with such a task, heating of the target area would be the primary mechanism for removal of organic matter. This means that surrounding areas would undergo some unavoidable heating and damage.

Srinivasan and Mayne-Banton in an article in Applied Physics Letters 41(6), 15 Sept. 1982, pp 576—578, described the self-developing photoetching of poly(ethylene terephthalate) films by far-ultraviolet excimer laser radiation.

It is one but not the exclusive object of this invention to provide for efficient removal of organic biological material without heating or thermal damage or pyrolysis or adverse effects to the areas of the material surrounding the area being irradiated.

Disclosure of the invention

In its broadest sense, this invention relates to the use of ultraviolet radiation of wavelengths less than 200 nm for etching or eroding biological organic material. The organic material can be selectively removed without undue heating and damage to the areas surrounding the area which is struck by the radiation. The mechanism by which the organic material is removed, or etched, is different from that of the prior art, and the geometry of the eching pattern can be completely defined by the ultraviolet beam.

In the technique of this invention, ultraviolet radiation of less than 200 nm wavelength has a very high efficiency for decomposing organic biological matter by electronic excitation of the constituent bonds of the organic matter, followed by bond breaking. The organic material is removed by ablative photodecomposition without heating or otherwise damaging the remaining organic material. This is initially a relatively linear photochemical effect, and inhomogeneities in the organic materials doe not effect the photo etching.

Although the attached claims do not cover methods for treatment of a human or animal body by surgery or therapy, this technique is also useful for many different types of uses and applications including surgical and dental applications. For example, damaged or unhealthy tissure can be removed from bones without damaging the bone itself and without traumatizing the remaining tissue. Also, skin lesions can be removed without traumatizing any of the surrounding skin. Healthy tissure can be cut or sectioned by the technique of this invention, without heating the edges of the cut. This also minimizes trauma. In addition to these exemplary types of applications, the invention can be used to treat decayed teeth, removing dental caries while leaving enamel and healthy dentine unaltered, in a possibly painless manner.

The source of ultraviolet radiation can be any known source as long as the radiation is or includes one or more wavelengths in the range less than 200 nm and as long as ablative photodecomposition occurs. Pulsed radiation of energy fluence at least about 10 mJ/cm²/pulse is preferable, but continuous radiation can also be used. A suitable ultra-violet wavelength source is an ArF excimer laser providing a pulsed output at 193 nm. Such lasers are commercially available.

Claim 1 is directed to a method for removing selected areas of a biological layer comprised of organic material, and is the subject of a disclaimer at the end of the claim, disclaiming from the scope of the claim methods for treatment of a human or animal body by surgery or therapy. Claim 2 is similar, and without the disclaimer, but specifies that the biological layer lacks human or animal life. Claim 3 is also similar but specifies that the layer is separated from a human or animal body. In support of these claims in will be apparent to the reader of this specification that the invention can be applied, for example, in post mortem examinations, and to non-viable tissues separated from a human or animal body. The invention has been applied for example to a sample of dead avian muscle tissue separated from the deceased body. The expression used in the claims "biological layer comprised of organic material" is intended to have its normal broad scope based on the meaning of "biology" as the science of life.

Brief description of the drawings

The Fig. illustrates schematically one type of suitable apparatus for carrying out this invention.

Best mode for carrying out the invention In the practice of this invention, ultraviolet radiation of wavelengths less than 200 nm is used to selectively remove organic material.

The radiation is applied either as pulsed radiation or as continuous wave radiation, and generally the pulsed radiation has a fluence greater than 10 mJ/cm²/pulse. Ultraviolet radiation in this wavelenth range does not burn organic materials such as human tissue; instead, it ablates the material, removing thin (micrometer) layers, layer by layer, for each pulse. In contrast, inorganic materials such as bone or tooth enamel are not photodecomposed by such radiation, at such energy densities.

Ultraviolet radiation having wavelengths less than 200 nm is capable of decomposing the organic material by electronically exciting the constituent bonds of the material, followed by bond-breaking and the production of volatile or gaseous materials which evaporate or escape from the surface. These photochemical reactions are particularly efficient for wavelengths less than 200 nm (i.e., vacuum ultraviolet radiation). In ablative photodecomposition, the broken fragments of biological matter require a larger volume than the unbroken chemical chains and "explode" from the biological matter, carrying away kinetic energy. The only products may have a molecular weight less than 100.

A suitable apparatus for carrying out the invention is shown in the Fig. It includes a source 10 of ultraviolet radiation of wavelengths less than 200 nm. A suitable source is an ArF excimer laser operating at a wavelength 193 nm. Such lasers are commercially available and are made by, for example, Lambda-Physik, W. Germany (a subsidiary of Coherent, Inc.). A specific laser with desirable properties for this application is the Lambda-Physik EMG-150 with an output of about 200 MJ/pulse, and a beam divergence of 200 microradians. These lasers routinely offer repetition rates of 60—100 pulses/second at an energy fluence greater than 200 millijoule/cm²/pulse. The typical pulse duration is about 10 nsec.

A casing 12 is used to contain the laser beam 14 (indicated by the dashed lines). In a preferred embodiment, casing 12 is vented with nitrogen gas to remove oxygen from the beam path, since oxygen absorbs radiation of 193 nm wavelength.

Casing 12 includes a shutter 16 which can be used to block the radiation beam, or allow it to pass. Also included within casing 12 is a 100% reflecting mirror 18, which is used to change the direction of the ultraviolet radiation 14. A lens 20 is optional, and can be used to focus the radiation beam onto a selected spot of the organic material 22. An aperture 24 is located in front of lens 20 to provide further collimation of the radiation before it strikes lens 20. Also, a mask 26 optionally can be located on or close to the organic material 22 in order to more fully define the incident ultra-violet radiation.

In an actual instrument, casing 12 could be part of a moveable arm having articulated joints so that the radiation beam could be moved about, the end piece of the instrument being held in the surgeon's hand similarly to the holding of a scalpel. The instrument can also be moved relative to the patient, under the control of an alignment apparatus.

For the exact design of a suitable flexible casing, reference is made to aforementioned U.S. Patent 3,769,963 which shows the use of an articulating arm and to aforementioned U.S Patent 3,821,510 which also shows a flexible laser-beam transmitting conduit that is capable of being held by hand. In addition to these references, aforementioned U.S. Patent 4,316,467 describes a technique for regulating the output energy of the laser in accordance with the absorption of the incident radiation. If the apparatus is to be stationary, movement of the beam steering mirror 18 in the directions indicated by arrow 27, can be used to scan the radiation beam over a portion of the organic material to be etched.

The following will detail some examples of the medical and dental applications of the above described apparatus. In a first application, bone surgery, ultraviolet radiation of wavelengths less than 200 nm is used to clean organic tissue from bone. In such surgery, it is usual to cut or scrape tissue from bone with scissors or scalpel. This prior art technique can traumatize the nearby healthy tissue resulting in swelling and unnecessary bleeding. To avoid these problems, ultraviolet light of wavelengths less than 200 nm and sufficient energy to ablatively photodecompose the tissue is focussed on the tissue to remove the tissue with great precision, without undesirable thermal effects. The tissue can be removed down to the bone without damaging the bone. This is because these wavelengths do not affect materials such as bone, which are many-fold less susceptible to ablative photoetching. Also, the inorganic bone surface is not affected by the laser energy fluence levels ($10$—$300$ mJ/cm$^2$/pulse) that effectively remove organic tissue.

In order to illustrate the advance of the present technique with respect to that of the prior art using lasers providing different wavelengths, grooves were cut into a piece of cartilage attached to a bone using, alternatively, ArF laser radiation at 193 nm and laser radiation from a frequency-doubled, pulsed Nd+ YAG laser. The ArF excimer laser (193 nm) delivered pulses at approximately 100 mJ/pulse at 10 pulses per second for approximately five seconds. This laser light was focussed with a cylindrical lens and irradiated a line approximately 30 mil long and 0.3 millimeter wide. The energy fluence was about 1000 mJ/cm$^2$/pulse. This radiation produced a groove approximately 150 micrometers deep, with sharp edges and a uniform depth.

The frequency-doubled Nd+ YAG laser delivered pulses at approximately 100 mJ/pulse at ten pulses per second for approximately five seconds. The wavelength was 532 nm, in the green portion of the visible spectrum. This laser was also focussed with cylindrical lens to an area comparable to that illuminated by the ArF laser. Thus, the energy fluences produced by the two lasers were comparable.

In the case of the Nd: YAG laser, the line which was produced in the cartilage was very burned, and was comprised of burned-looking islands running approximately parallel to the clean groove which resulted from irradiation with the ArF laser. While the ArF laser at 193 nm cleanly removes cartilage, the frequency-doubled Nd: YAG laser charred the cartilage and produced raised carbonized islands.

Far-UV radiation in the wavelength range below 200 nm causes the cartilage tissue to undergo linear photochemistry in the first step of this process, and material inhomogeneities in the organic tissue are unimportant. In contrast with this, the photochemical effects are very nonlinear when visible wavelengths are used and inhomogeneities in the organic tissue play an important role in determining where the charred islands occur.

Another application of the above described apparatus is the treatment of various skin defects. For example, port-wine scars (hemangiomas) and other types of birthmarks can be selectively removed by a bloodless surgical procedure. Here, the far-UV light is used to carefully remove thin layers of skin without undesirable thermal side effects and without undue bleeding.

For example, an ArF laser of the type shown can be used to excise skin cancer, remove port-wine scars, and remove "age" spots. Another application is the treatment of a common form of skin cancer, termed basal cell carcinoma. This type of cancer is often caused by damage from short wavelength ultraviolet light between 280—315 nm, such as that produced by sunlight.

This wavelength region of sunlight produces sunburn and burning of the basal cell layer located between the epidermis and the dermis, and can result in basal cell carcinoma. When basal cell layer burning occurs, sometimes local areas undergo uncontrolled growth, which is the carcinoma. Longer ultraviolet wavelengths, between 315 and 400 nm, cause pigment darkening or "tanning" in the epidermis. This mechanism helps to protect the basal cell layer from burning.

A common technique for removing basal cell carcinoma involves scraping the skin at and around the lesion in order to take off layers of skin until all the malignant cells are removed. The dermatologist uses personal experience and "feel" as he scrapes the skin in layers, to determine when to stop scraping. In this procedure, the surrounding skin is often damaged and can become scarred in an unsightly manner over a larger area than is desirable.

When far-ultraviolet light surgery is used, the carcinoma can be removed with a minimum of damage to healthy skin. The dermatologist uses the radiation beam to remove thin layers, layer by layer, and either uses his experience to determine when to stop, or uses some other chemical type of method. For example, the carcinoma may fluoresce differently than healthy skin under low-level long wavelength ultraviolet radiation. Since the skin removal process by ultraviolet radiation of wavelengths less than 200 nm is clean (i.e., no bleeding or scarring), it is easy to view the remaining tissue, unobscured by the roughened skin surface or by blood. This makes its easier to be able to tell when to stop removal of tissue.

Another application of this apparatus is in the field of dental medicine. Far-ultraviolet radiation of wavelengths less than 200 nm can be used to remove decay from teeth without damaging the surrounding enamel. As is known, teeth have an outside protective layer is calcium-based enamel. Decay is caused by bacterial action of food particles, particularly those containing sugar. This bacterial action produces lactic acid, which enters pores and cracks in the enamel and destroys the enamel to reach the organic dentine. A decay action then continues into the interior of the tooth toward the nerve. In the prior art, decay is treated by removing all of the decayed dentine and enamel, and filling the cavity with amalgam, gold, or some other nontoxic durable filler. The tooth is cleaned out with a mechanical drill, and much healthy enamel and dentine is also removed. The friction from the drilling produces heat and can be quite painful.

In contrast with this prior art technique, far-ultraviolet radiation of wavelengths less than 200 nm can be used to ablatively remove decay. In this technique the ultraviolet beam is focussed upon the decayed area of the tooth and photoetches the decay matter, producing volatile products which escape. The enamel will not be damaged, and the decayed dentine will be selectively eroded with no undesirable thermal side effects. This treatment may be entirely painless and can be used to limit the amount of area that is photoetched to exactly the area containing the decay. The apparatus shown can be used as a drill to selectively cut away the decayed dentine without "touching" the enamel which is to remain.

An additional use of this apparatus is in periodontal surgery. In this type of surgery, the gum tissue will be selectively eroded or cut, leaving the tooth enamel undamaged.

Another application for this apparatus is in cutting or sectioning healthy organic tissue without damaging the edges of the cut. Since undamaged tissue edges heal more neatly and safely than ragged edges or scarred tissues, the chance of infection or the presence of an unsightly scar is reduced.

In order to further demonstrate the degree of control over the etching process and the absence of heating affects, a sample of human hair was irradiated through a metal mask. Ablative photodecomposition of the hair was achieved by irradiating the human hair sample through a metal mask with 193 nm laser radiation. The energy fluence of the laser pulse was 250 kJ/cm$^2$/pulse. The rate of removal of the hair material was approximately 400Å per pulse, which is about three-fold greater than the rates which have been realized when etching synthetic polymers with this radiation (as described in out European Application EP—A—0098917). An enlarged wiew of the ablated material showed n evidence of thermal damage. In this irradiation, the hair was etched to controlled depths to provide rectangular grooves therein having sharply defined edges and uniform depth in each groove.

In the practice of this invention, organic biological material can be photodecomposed in an ablative process that produces volatile products that escape. The ultraviolet radiation source can be any source providing radiation of wavelengths less than 200 nm. The threshold energy flux for pulse radiation is about 10 mJ/cm$^2$/pulse. In this process, approximately 0.2 micrometers of organic tissue or other matter are removed by each radiation pulse having at least 10 mJ/cm$^2$/pulse. The pulse width of the incident ultraviolet radiation is not critical to the process and, in fact, continuous radiation may also be used.

The photodecomposition of the organic biological matter in this process is characterized by the absorption of a very large proportion (approximately 95%) of the incident photons in a thin (less than 2700 Å) layer of the organic material, and by the breaking of a large number of protein bonds in the material with a high (10—100%) quantum yield. Ejection of photolyzed material as small volatile molecules occurs into the surrounding atmosphere. These volatile or gaseous compounds typically have a low (less than 100) molecular weight. The irradiated surface is photoetched in a pattern that is defined by the light.

While it has been mentioned that both continuous wave radiation and pulsed radiation can be used in the practice of this invention, it may be that continuous wave radiation will be quite impractical. In the situation where continuous wave radiation is used, the bonds in the biological layer of organic matter may be broken but may recombine and deposit again if the process proceeds too slowly. Using a pulsed radiation source means that a large amount of energy can be delivered in a vary small amount of time. When this occurs, the bonds are broken in the biological layer in a short amount of time, pressure is built up, and volatile products blow off. This is the mechanism of ablative photodecomposition (APD), which requres that the broken fragments be produced in a small volume in a sufficiently short time that they blow off due to the pressure buildup. The radiation source must provide this type of power density of ADP to occur.

In the first step, the photochemistry is linear, the bonds being broken by the incident radiation. However, the blow-off of volatile products is a nonlinear function of the rate at which the energy is introduced into the biological layer. In the example given previously, a minimum energy flux of about 10 mJ/cm$^2$/pulse is sufficient to provide ablative photochemistry in which volatile products are blown off after the pulse (which is about 10 nsec. wide) is applied.

The wavelength of the incident ultraviolet radiation is chosen to be less than 200 nm, and generally extends down to about 100 nm. In the spectroscopic art, this is termed the vacuum ultraviolet range, and generally comprises those wavelengths which begin to be absorbed in air. For example, oxygen begins to absorb radiation at about 200 nm, and this is why the apparatus shown is vented with nitrogen. As the wavelength decreases, more and more absorption will occur by different gases.

Upon absorption of radiation in the wavelength range 100—200 nm, only a thin layer of the tissue is ablatively photodecomposed, and the radiation will not penetrate and damage other portions of the tissue. In contrast with this, longer wavelength radiation will produce burning and will not be characterized by ablative photodecomposition.

In addition to the reasons described above for choosing the appropriate wavelength range, another practical reason exists with respect to the apparatus. It is known that lithium fluoride can be used as a transmission window located adjacent to the laser radiation source.

Lithium fluoride has the shortest wavelength of transmission, and will cut off at approximately 110 nm. That is, at wavelengths shorter than 110 nm, the lithium fluoride will not be transparent.

Thus, for wavelengths less than 110 nm, a lithium fluoride window cannot be used, and the laser output would have to be passed through a vacuum chamber in order to prevent large amounts of absorption of the radiation. This would be a complex and costly apparatus. Also, no optical fibre is known which can transmit radiation at wavelengths less than 110 nm.

Thus far, an ablative photodecomposition process has been described in which no heat effects are produced. That is, the photochemistry is such that the energy in the incident ultraviolet radiation is transmitted to the kinetic energy of the volatile products leaving the biological layer that is irradiated. The energy which is present in the ultraviolet beam is not transmitted as heat to the biological layer. This has been confirmed by measurements which look at the morphology of the material. Additionally, it is an effect which can be readily felt. As an example, a person can place his hand in the path of ultraviolet radiation having wavelengths less than 200 nm, and experience no pain. Only a small "pressure" is felt when the volatile gases are blown off, the pressure being a recoil when these gases blow off.

While the ablative photodecomposition intended by the process of the present invention does not lead to a noticeable heat buildup in the biological layer, it is obvious that heat will begin to occur if more and more energy is applied. That is, when the amount of energy supplied is greater than the amount which can be carried away by the volatile byproducts, some heating will begin to occur.

The minimum energy flux for producing ablative photodecomposition of these biological layers is about 10 mJ/cm$^2$/pulse, and the maximum energy for practical purposes is that which begins to cause detrimental heating and other effects similar to those which are seen when radiation of wavelengths greater than 200 nm is applied. This maximum amount of input energy flux depends upon the particular type of biological layer being photodecomposed in accordance with the present invention. Generally, it is desired that no significant amount of heating should occur in either the medical or dental applications of the apparatus. However, in its broadest sense the invention relates to ablative photodecomposition of biological layers at wavelengths less than 200 nm. For pulsed radiation, this effect begins to occur if the input energy flux is at least about 10 mJ/cm$^2$/pulse.

As a corollary to the fact that heat is not produced when the input energy flux is not unduly great, it has been noted that a pulsed beam of radiation from a YAG laser operating at the same power level will cause the sensation of pain to a human, while ultraviolet radiation at wavelengths less than 200 nm will not cause this sensation of pain. Of course, the reason is straightforward and is due to the difference between ablative photodecomposition in accordance with the present invention and decomposition resulting from a burning effect as is experienced in the prior art.

Using ultraviolet radiation of wavelengths less than 200 nm has yielded ablative photodecomposition of biological matter without noticeable pyrolytic heat effects (i.e., chemical changes induced by heat) at energy fluxes up to about 1000 mJ/cm$^2$/pulse, although this is not necessarily an upper limit. Any type of medical or dental application can be undertaken with the above described apparatus using ablative photodecomposition at wavelengths less than 200 nm. While the invention has been particularly described with respect to certain embodiments and applications, it will be readily apparent to those of skill in the art that other applications can be made without departing from the scope of the invention which is defined in the attached claims.

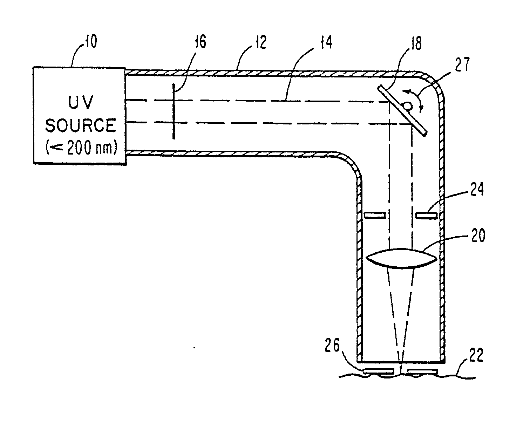

**Claims**

1. A method for removing selected areas of a biological layer comprised of organic material, including the steps of:

selecting a desired area of said biological layer, and

irradiating said desired area with ultraviolet radiation having wavelength(s) less than 200 nanometers and energy fluences sufficient to cause ablative photodecomposition to said biological layer,

subject to a disclaimer to methods for treatment of a human or animal body by surgery or therapy.

2. A method as claimed in claim 1 in which the biological layer is lacking in human or animal life.

3. A method as claimed in claim 1 in which the biological layer is separated from a human or animal body.

**Patentansprüche**

1. Verfahren zur Entfernung ausgewählter Bereiche einer aus organischem Material aufgebauten biologischen Schicht, mit den Verfahrensschritten des

Auswählens eines gewünschten Bereichs der biologischen Schicht, und

Bestrahlens des gewünschten Bereichs mit Ultraviolettstrahlung mit (einer) Wellenlänge(n) von weniger als 200 Nanometern und Energieflüssen, die ausreichen, eine ablative photochemische Zersetzung an der biologischen Schicht zu bewirken,

unter einem Disclaimer von Verfahren zur Behandlung eines menschlichen oder tierischen Körpers durch Chirurgie oder Therapie.

2. Verfahren nach Anspruch 1, bei welchem die biologische Schicht in menschlichem oder tierischem Leben fehlt.

3. Verfahren nach Anspruch 1, bei welchem die biologische Schicht von einem menschlichen oder tierischen Körper getrennt ist.

**Revendications**

1. Procédé pour éliminer des zones sélectionnées d'une couche biologique constituée par une substance organique, incluant les étapes opératoires consistant à:

choisir une zone désirée de ladite couche biologique, et

irradier ladite zone désirée avec un rayonnement ultraviolet possédant une (des) longueur(s) d'onde inférieure(s) à 200 nanomètres et des fluences énergétiques suffisantes pour réaliser un enlèvement par photodécomposition dans ladite couche biologique,

sous réserve de renonciation à des méthodes de traitement chirurgical ou thérapeutique d'un corps humain ou animal.

2. Procédé selon la revendication 1, selon lequel la couche biologique n'est le siège d'aucune vie humaine ou animale.

3. Procédé selon la revendication 1, selon lequel la couche biologique est séparée d'un corps humain ou animal.